(19) **Europäisches Patentamt / European Patent Office / Office européen des brevets**

(11) **EP 2 309 462 B1**

(12) **FASCICULE DE BREVET EUROPEEN**

(45) Date de publication et mention de la délivrance du brevet:
**23.08.2017 Bulletin 2017/34**

(51) Int Cl.:
*G06T 7/00* ^(2017.01)    *G06T 11/00* ^(2006.01)

(21) Numéro de dépôt: **11151762.9**

(22) Date de dépôt: **11.06.2001**

(54) **Procédé et dispositif d'imagerie radiographique pour la reconstruction tridimensionnelle à faible dose d'irradiation.**

Verfahren und Vorrichtung zur dreidimensionalen Rekonstruktion von Röntgenbilder mit niedriger Strahlungsdosis

Radiographic imaging method and device for three-dimensional reconstruction with low dose of irradiation

(84) Etats contractants désignés:
**CH DE GB IT LI SE**

(30) Priorité: **23.06.2000 FR 0008123**

(43) Date de publication de la demande:
**13.04.2011 Bulletin 2011/15**

(62) Numéro(s) de document de la (des) demande(s) initiale(s) en application de l'article 76 CBE:
**01401511.9 / 1 168 249**

(73) Titulaire: **EOS Imaging**
**75011 Paris (FR)**

(72) Inventeurs:
 • **Dorion, Irène**
  **75011 PARIS (FR)**
 • **Desaute, Pascal**
  **75020 PARIS (FR)**
 • **Charpak, Georges**
  **75005 PARIS (FR)**
 • **Skalli, Wafa**
  **75013 PARIS (FR)**
 • **Veron, Stéphane**
  **92130 ISSY-LES-MOULINEAUX (FR)**
 • **Mitton, David**
  **94270 LE KREMLIN BICETRE (FR)**
 • **De Guise, Jacques A.**
  **MONTREAL Québec H2W2L6 (CA)**
 • **Landry, Champlain**
  **MONTREAL Québec H2J3K8 (CA)**

(74) Mandataire: **Cabinet Plasseraud**
 **66, rue de la Chaussée d'Antin**
 **75440 Paris Cedex 09 (FR)**

(56) Documents cités:
**EP-A2- 0 747 728    US-A- 5 951 475**

 • **D.MITTON ET AL: "3D reconstruction method from biplanar radiography using non-stereocorresponding points and elastic deformable meshes", MEDICAL & BIOLOGICAL ENGINEERING & COMPUTING 2000, vol. 38, mars 2000 (2000-03), pages 133-139, XP000913390,**
 • **LAPORTE S ET AL: "A biplanar reconstruction method based on 2D and 3D contours: application to the distal femur", COMPUTER METHODS IN BIOMECHANICS AND BIOMEDICAL ENGINEERING, INFORMA HEALTHCARE/ ABINGDON : TAYLOR & FRANCIS, 1997-, GB, vol. 6, no. 1, 1 January 2003 (2003-01-01) , pages 1-6, XP009147936, ISSN: 1025-5842, DOI: 10.1080/1025584031000065956**
 • **LAVALLEE S ET AL: "RECOVERING THE POSITION AND ORIENTATION OF FREE-FORM OBJECTS FROM IMAGE CONTOURS USING 3D DISTANCE MAPS", TRANSACTIONS ON PATTERN ANALYSIS AND MACHINE INTELLIGENCE, IEEE, PISCATAWAY, USA, vol. 17, no. 4, 1 April 1995 (1995-04-01), pages 378-390, XP000499568, ISSN: 0162-8828, DOI: 10.1109/34.385980**

EP 2 309 462 B1

## Description

La présente invention est relative aux procédés et dispositifs d'imagerie radiographique pour la reconstruction tridimensionnelle à faible dose d'irradiation.

Des procédés de reconstruction en trois dimensions ont été divulgués notamment par Abdel-Aziz et al. ("Direct linear transformation from comparator coordinates into object space coordinates in close range photogrammetry", Proc. ASP/UI Symp. Close Range Photogrammetry, Urbana, Illinois, USA, 1971) et Marzan ("Rational design for close range photogrammetry", PhD thesis, Department of Civil Engineering, University of Illinois, Urbana-Champaign, USA, 1976).

Dans ces procédés, tous les repères de contrôle sont des points de contrôle stéréo-correspondants, et on positionne ces repères dans l'espace au moyen d'un algorithme dit "transformation linéaire directe" (DLT), utilisé notamment par André et al. ("Optimized vertical stereo base radiographie setup for the clinical three-dimensional reconstruction of the human spine", J. Biomech., 27, pp 1023-1035, 1994). Par ailleurs, dans ces procédés connus, on met en oeuvre une étape de krigeage consistant en une interpolation/extrapolation du modèle générique de l'objet à observer, qui donne des positions estimées d'un grand nombre de repères du modèle à trois dimensions de l'objet à observer en fonction des coordonnées mesurées des repères de contrôle stéréo-correspondants et en fonction de la géométrie du modèle générique. Cette étape de krigeage a été décrite notamment par Trochu ("A contouring program based on dual kriging interpolation", Eng. Comput. 9, pp 160-177, 1993).

Ces procédés connus présentent l'avantage de permettre la réalisation d'un modèle tridimensionnel du ou des objets à observer, tout en permettant de réduire l'émission de rayonnements ionisants vers le champ d'observation par rapport à une information tridimensionnelle basée sur la reconstruction de coupes scanner telles que pratiquées dans les instruments actuels. Le modèle tridimensionnel peut ensuite être affiché sous différents angles de vue, par exemple sur un écran d'ordinateur.

Mais ces procédés souffrent d'un manque de précision et sont mal adaptés pour examiner convenablement un champ d'observation étendu tel que par exemple l'ensemble de la colonne vertébrale d'un patient.

Par ailleurs, MITTON et al. (Medical & Biological Engineering & Computing 2000, Vol. 38, p 133-139) ont décrit un procédé de reconstruction tridimensionnelle qui utilise des repères non stéréo-correspondants en plus des repères stéréo-correspondants, pour reconstruire une image à trois dimensions d'une vertèbre isolée, à partir de deux images radiographiques de cette vertèbre prises successivement sous deux angles différents.

Ce procédé n'est toutefois pas adapté pour une utilisation médicale, où il est nécessaire d'avoir une plus grande facilité de mise en oeuvre, une meilleure précision notamment sur des champs d'observation étendus, et/ou une plus grande rapidité de mise en oeuvre.

La présente invention a notamment pour but de pallier ces inconvénients.

A cet effet, selon l'invention, on prévoit un procédé d'imagerie radiographique pour la reconstruction tridimensionnelle à faible dose d'irradiation, adapté pour calculer un modèle à trois dimensions d'au moins un objet prédéterminé à observer dans un champ d'observation, dans lequel :

1) on repère sur au moins deux images radiographiques des lignes de contour correspondant à des limites de l'objet et/ou à des lignes de plus grande densité optique à l'intérieur desdites limites,
2) on crée un modèle générique recalé en adaptant la taille d'un modèle générique correspondant audit objet et la position dudit modèle générique pour que les projections respectives du modèle générique recalé à partir de deux sources radioactives correspondent sensiblement aux deux images radiographiques,
3) on sélectionne des repères du modèle générique recalé dont les projections sur au moins une des images radiographiques à partir de la source radiographique correspondante sont les plus proches des lignes de contour repérées au cours de l'étape 1),
4) on définit une surface enveloppe formée par des rayons issus de chaque source radiographiques et ayant contribué à générer lesdites lignes de contour,
5) on détermine certains repères du modèle générique recalé correspondant à des surfaces dudit modèle générique recalé, qui sont tangentes auxdites surfaces enveloppes,
6) on détermine la position géométrique de chaque repère déterminé à l'étape 5) par projection dudit repère sur la surface enveloppe correspondante,
7) on calcule la forme à trois dimensions d'un modèle représentant ledit objet par déformation du modèle générique recalé de façon à maintenir la coïncidence des repères du modèle générique déformé avec la position spatiale précédemment déterminée des repères et de façon que ledit modèle calculé suive une forme la plus proche possible d'une isométrie du modèle générique.

Le champ d'observation mentionné ci-dessus peut comprendre notamment le rachis, le bassin, ou encore le genou d'un patient, ou plus généralement être constitué par tout ou partie du squelette du patient. Dans ces différents cas, les objets à observer peuvent être constitués notamment par les os du patient compris dans le champ d'observation ainsi que la durée des opérations de prise de vue.

Grâce aux dispositions susmentionnées, on obtient une bonne précision de la reconstruction à trois dimensions, éventuellement pour des champs d'observation étendus, et ce en limitant la dose de rayonnements émise vers le champ d'observation.

Cette précision peut-être obtenue grâce à la simultanéité des deux prises de vues, et grâce à la prise de vue par balayage qui améliore la précision dans la direction du balayage notamment pour les champs d'observation étendus.

Dans des modes de réalisation préférés du procédé selon l'invention, on peut éventuellement avoir recours en outre à l'une et/ou à l'autre des dispositions suivante :

- au cours de l'étape 7), on travaille sur l'ensemble des points du modèle générique ;
- (a) prendre au moins deux images radiographiques à deux dimensions du champ d'observation, respectivement selon deux directions de prise de vue non parallèles ;
- au cours de l'étape (a), les deux images radiographiques sont prises simultanément, par balayage, en déplaçant en synchronisme, dans une même direction de translation non parallèle aux directions de prises de vues, au moins une source radioactive émettant deux faisceaux de rayons ionisants respectivement dans les deux directions de prise de vue ;
- les deux directions de prise de vue sont perpendiculaires l'une à l'autre ;
- on fait émettre par chacune des sources radioactives un faisceau de rayonnements ionisants dans un plan perpendiculaire à la direction de translation ;
- les deux faisceaux de rayons ionisants sont émis respectivement par deux sources radioactives.

Par ailleurs, l'invention a aussi pour objet un produit programme d'ordinateur adapté pour mettre en oeuvre le procédé lorsqu'il est mis en oeuvre sur une machine programmable.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue schématique d'un appareil de radiographie selon une forme de réalisation de l'invention, permettant d'effectuer simultanément une prise de vue de face et une prise de vue de profil du patient,
- la figure 2 est une vue schématique en perspective d'une vertèbre d'un patient examiné au moyen de l'appareil de la figure 1,
- les figures 3 et 4 sont respectivement des vues de profil et de face de la vertèbre de la figure 2, schématisant une partie des vues de profil et de face obtenues au moyen de l'appareil de la figure 1,
- et la figure 5 est une vue en perspective représentant un modèle à trois dimensions de la colonne vertébrale et du bassin du patient examiné au moyen de l'appareil de la figure 1, ce modèle étant calculé à partir des vues de profil et de face prises au moyen de l'appareil de la figure 1.

Sur les différentes figures, les mêmes références désignent des éléments identiques ou similaires.

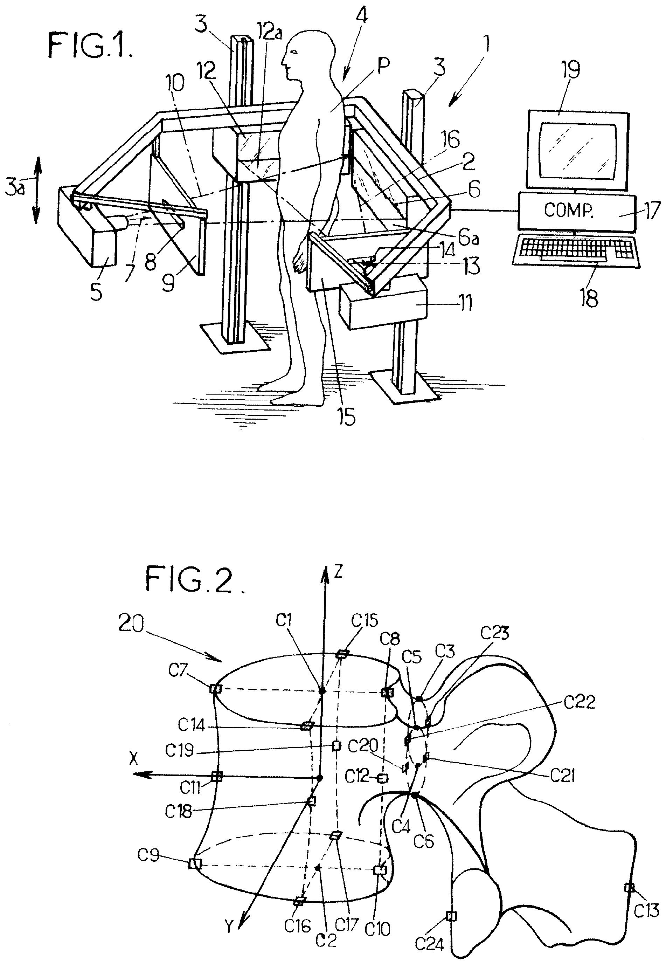

La figure 1 représente un dispositif radiographique 1 pour la reconstruction tridimensionnelle, comportant un bâti mobile 2 déplaçable verticalement de façon motorisée sur des guides verticaux 3, dans une direction de translation 3a.

Ce bâti entoure un champ d'observation 4 dans lequel peut prendre place un patient P debout. On peut ainsi observer la position des os du squelette de ce patient en station debout, ce qui est essentiel notamment pour les patients atteints de scoliose.

Le bâti mobile 2 porte une première source radioactive 5 et un premier détecteur 6 qui est disposé face à la source 5 au-delà du champ 4, et qui comporte une ligne horizontale 6a de cellules de détection. Le détecteur 6 peut par exemple être un détecteur gazeux sensible aux basses doses de rayonnements, par exemple tel que décrit dans le document FR-A-2 749 402 ou FR-A-2 754 068. Bien entendu, d'autres types de détecteurs, gazeux ou non, pourraient éventuellement être utilisés dans le cadre de la présente invention.

La source radioactive 5 est adaptée pour émettre des rayons ionisants, notamment des rayons X, dans une direction de prise de vue 7 antéro-postérieure par rapport au patient P, en traversant une fente horizontale 8 ménagée dans un réticule 9 tel qu'une plaque métallique, pour générer un faisceau horizontal 10 de rayonnements ionisants dans le champ d'observation 4.

Par ailleurs, le bâti mobile 2 porte également une deuxième source radioactive 11 similaire à la source 5 et un deuxième détecteur 12 similaire au détecteur 6, qui est disposé face à la source 11 au-delà du champ 4, et qui comporte une ligne horizontale 12a de cellules de détection.

La source radioactive 11 est adaptée pour émettre des rayons ionisants, dans une direction de prise de vue latérale 13 par rapport au patient P, en traversant une fente horizontale 14 ménagée dans un réticule 15 tel qu'une plaque métallique, pour générer un faisceau horizontal 16 de rayonnements ionisants dans le champ d'observation 4.

On notera que les sources radioactives et les détecteurs pourraient le cas échéant être en nombre supérieur à 2, et que les directions de prises de vue de ces différentes sources radioactives pourraient le cas échéant ne pas être perpendiculaires entre elles ni horizontales.

Les deux détecteurs 6, 12 sont reliés à un micro-ordinateur 17 ou autre système électronique de commande, équipé :

- d'une interface d'entrée comprenant au moins un clavier et généralement une souris (non représentée),

- et d'une interface de sortie comprenant au moins un écran 19 et généralement une imprimante (non représentée).

Le micro-ordinateur 17 peut également être relié aux moyens d'entraînement motorisés (non représentés) contenus dans les guides 3 et aux sources 5, 11, de façon à commander le déplacement vertical du bâti 2 et l'émission des rayonnements ionisants.

Le dispositif qui vient d'être décrit fonctionne comme suit.

Au moyen du micro-ordinateur 17, on fait d'abord prendre deux images radiographiques du patient P, en faisant balayer le champ d'observation 4 par les faisceaux 10, 16 de rayonnements ionisants sur la hauteur correspondant à la zone du patient à observer, par exemple le rachis et le bassin, voire l'ensemble du squelette (à cet effet, le bâti est de préférence déplaçable sur une hauteur d'au moins 70 cm, voire supérieure à 1 m).

Au cours de ce mouvement, on enregistre dans la mémoire du micro-ordinateur 17 deux images radiographiques numériques, par exemple respectivement antéro-postérieure et latérale de la partie examinée du patient, lesquelles images peuvent être visualisées sur l'écran 19 du micro-ordinateur.

Chacune de ces images comprend généralement plusieurs objets prédéterminés à examiner, par exemple des vertèbres 20 telles que celle représentée schématiquement sur la figure 2.

Pour chacun de ces objets à examiner, le micro-ordinateur 17 a en mémoire un modèle générique à trois dimensions qui correspond à une forme moyenne de l'objet en question, lequel modèle générique est élaboré à l'avance par des méthodes statistiques en analysant un grand nombre d'objets similaires.

Lors de l'affichage des images radiographiques sur l'écran 19 du micro-ordinateur 17, le praticien peut par exemple indiquer au micro-ordinateur, notamment au moyen du clavier 18 ou de la souris, le type de chaque objet à examiner visible sur lesdites images, de façon que le micro-ordinateur 17 détermine le modèle générique correspondant à cet objet.

Par ailleurs, les modèles génériques utilisés pourraient également être constitués par des modèles préalablement réalisés par imagerie médicale sur le patient : dans ce cas, le procédé selon l'invention peut permettre par exemple de suivre l'évolution ultérieure du patient par des moyens plus simples, moins coûteux et émettant moins de radiations que les moyens d'imagerie tridimensionnelle classiques.

Le modèle générique de chaque objet, par exemple de chaque vertèbre 20 d'un squelette humain, comprend :

- les coordonnées d'une pluralité de repères de contrôle, notamment des points correspondant à des repères singuliers de cette vertèbre,

- et les coordonnées d'un grand nombre d'autres repères de l'objet en question, par exemple au nombre d'environ 200 ou plus.

Ces coordonnées peuvent être exprimées par exemple dans un référentiel local X, Y, Z. Dans l'exemple considéré, l'axe Z correspond à la direction "axiale" de la colonne vertébrale, l'axe X est déterminé de façon à définir avec l'axe Z le plan antéro-postérieur de la vertèbre 20, l'axe Y étant perpendiculaire aux axes X, Z susmentionnés. De plus, l'origine O du référentiel X, Y, Z est disposée au milieu des deux faces d'extrémité axiales de la partie principale "tubulaire" de la vertèbre, l'origine O étant par ailleurs positionnée pour que l'axe Z traverse la face axiale supérieure de la partie principale de la vertèbre en un repère C1 tel que la distance de ce repère C1 à l'extrémité avant C7 de ladite face axiale soit égale à environ 2/3 de la distance totale entre les extrémités avant C7 et arrière C8 de la section antéro-postérieure de ladite face axiale supérieure.

Les différents repères de contrôle C1-C25 susmentionnés se répartissent en deux catégories :

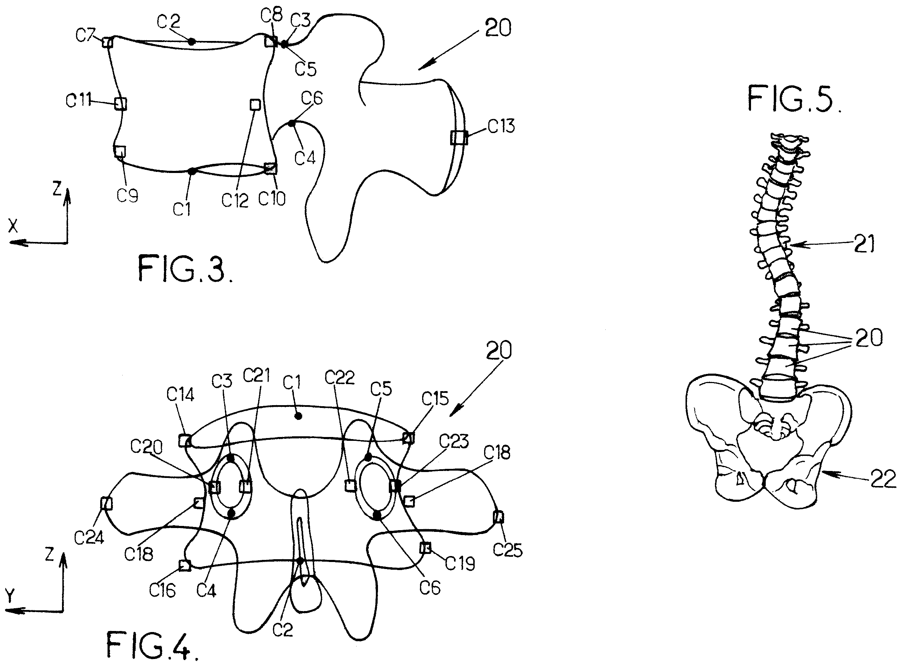

- des repères de contrôle "stéréo-correspondants" C1-C6, visibles et identifiables à la fois sur l'image radiographique latérale et sur l'image antéro-postérieure, ces repères étant au nombre de 6 dans l'exemple considéré (voir figures 3 et 4),
- et des repères de contrôle "non stéréo-correspondants" C7-C25, visibles et identifiables sur une seule image, ces repères étant au nombre de 19 dans l'exemple considéré.

Le praticien identifie ces différents repères de contrôle pour chaque objet à examiner (par exemple les vertèbres et le bassin) sur chaque image radiographique, par exemple en "marquant" ces repères à l'écran 19 par sélection au moyen de la souris et/ou du clavier. De plus, les deux images sont calibrées, de façon à pouvoir mesurer précisément la position de chaque repère de ces images dans un référentiel commun.

Ensuite, on détermine une position géométrique de chaque repère de contrôle de chaque objet, dans un référentiel à trois dimensions, par exemple le référentiel X, Y, Z susmentionné ou un référentiel commun à l'ensemble des objets à examiner.

La position des repères de contrôle stéréo-correspondants C1-C6 est directement calculée à partir de la mesure de la position de ces points sur les deux images.

De plus, la position géométrique de chaque repère de contrôle non stéréo-correspondant C7-C25 dans le référentiel à trois dimensions est estimée à partir du modèle générique, en déplaçant chaque repère de contrôle stéréo-correspondant C1-C6 du modèle générique jusqu'à sa position mesurée, et en déplaçant les repères de contrôle non stéréo-correspondants C7-C25 du modèle générique, chacun sur une droite joignant :

- d'une part, la source radioactive 5, 6 à l'origine de l'image radiographique où une projection de ce repère de contrôle non stéréo-correspondant est visible et identifiable,
- et d'autre part, ladite projection de ce repère sur l'image radiographique,

les repères de contrôle non stéréo-correspondants étant ainsi déplacés jusqu'à des positions respectives qui minimisent la déformation globale du modèle générique de l'objet à observer.

En particulier, on peut minimiser ladite déformation en minimisant (par exemple au moyen d'une méthode de gradient) la valeur de la somme quadratique :

$$ S = \frac{1}{2}.\sum_{i=1}^{m} k_i.(x_i - x_{i0})^2 \ , $$

ou plus généralement $S = \lambda.\sum_{i=1}^{m} k_i.(x_i - x_{i0})^2$ , où

$\lambda$ est un coefficient constant prédéterminé, m est un nombre entier non nul représentant un nombre de ressorts fictifs qui relient chaque repère de contrôle du modèle générique à d'autres repères de contrôle, $k_i$ est un coefficient de raideur prédéterminé du ressort fictif d'indice i, $x_{i0}$ est la longueur du ressort fictif d'indice i dans le modèle générique non déformé, et $x_i$ est la longueur du ressort fictif d'indice i dans le modèle générique déformé.

Enfin, on calcule la forme à trois dimensions d'un modèle représentant la vertèbre 20 du patient, le modèle calculé étant obtenu par déformation du modèle générique de façon à maintenir la coïncidence des points de contrôle du modèle générique déformé avec la position spatiale précédemment déterminée des points de contrôle et de façon que ledit modèle calculé suive une forme la plus proche possible d'une isométrie du modèle générique, en travaillant cette fois sur l'ensemble des points de modèle générique.

En particulier, l'obtention du modèle à trois dimensions de chaque objet à examiner peut être obtenu par le procédé connu de krigeage ("kriging").

Après le calcul du modèle à trois dimensions des différents objets à examiner, le micro-ordinateur 17 peut assembler la totalité des modèles à trois dimensions des différents objets à examiner, en fonction de la position de ces différents modèles dans un référentiel absolu commun à tous ces objets, de façon à obtenir un modèle à trois dimensions comprenant par exemple l'ensemble du rachis 21 du patient et le bassin 22 de ce patient, comme représenté sur la figure 5.

Une fois élaboré, ce modèle à trois dimensions peut être présenté sur l'écran 19 du micro-ordinateur, ou imprimé, sous l'angle de vision voulu. Ce modèle d'ensemble peut également être mis en mouvement à l'écran en fonction des commandes du praticien.

Le praticien dispose ainsi d'un outil efficace d'examen pouvant servir à l'imagerie de toute partie notamment osseuse ou cartilagineuse du corps humain ou animal, et utile notamment pour le diagnostic des scolioses ou pour le suivi pré ou post-opératoire lors d'interventions chirurgicales.

Bien entendu, on peut également calculer certains indices cliniques prédéterminés liés soit à la géométrie de l'ensemble examiné, soit le cas échéant à la composition ou la densité des objets à examiner, estimées à partir des images radiographiques (cas de l'ostéoporose par exemple).

On notera que le dispositif radiographique 1 pourrait le cas échéant être adapté pour l'examen d'un patient couché, ce qui peut s'avérer indispensable dans le domaine de la traumatologie. Dans ce cas, le patient P serait couché sur une table support, les faisceaux de rayons ionisants 10, 16 seraient chacun dans un plan vertical, et les sources 5, 11 se déplaceraient horizontalement avec les détecteurs 6, 12.

Par ailleurs, il va de soi que dans tous les cas, le dispositif radiographique 1 peut être utilisé également en radiographie à deux dimensions, en plus de son utilisation en imagerie tridimensionnelle.

On notera que le dispositif selon l'invention pourrait le cas échéant être utilisé dans des applications de radiologie non médicale.

Par ailleurs, au lieu d'utiliser des repères de contrôle C1-C25 définis à l'avance sur chaque modèle générique, il serait possible de déterminer et de positionner dans l'espace les repères de contrôle à partir de lignes de contour de l'objet à observer visibles sur l'une ou l'autre des deux images radiographiques.

A cet effet, on pourrait en particulier procéder comme suit :

- on repère sur chaque image radiographique des lignes de contour correspondant à des limites de l'objet observé et/ou à des lignes de plus grande densité optique à l'intérieur desdites limites,
- on crée un modèle générique recalé en adaptant la taille du modèle générique et la position de ce modèle générique dans le référentiel X, Y, Z pour que les projections respectives du modèle générique recalé à partir des deux sources radioactives 5, 11 correspondent sensiblement aux deux images radiographiques,
- on sélectionne des repères du modèle générique dont les projections sur au moins une des images radiographiques à partir de la source radioactive correspondante, sont les plus proches des lignes de contour repérées au cours de l'étape (b),
- on définit une surface enveloppe formée par des rayons issus de chaque source radioactives et ayant contribué à générer lesdites lignes de contour des images radiographiques,
- on détermine certains repères du modèle générique recalé correspondant à des surfaces dudit modèle générique recalé, qui sont tangentes auxdites surfaces enveloppes, les repères du modèle générique recalé ainsi déterminés correspondant aux repères de contrôle,

- on détermine la position géométrique de chaque repère de contrôle par projection dudit repère de contrôle sur la surface enveloppe correspondante,

- puis on procède par exemple comme décrit précédemment pour reconstituer un modèle à trois dimensions de l'ensemble de l'objet à observer, notamment par krigeage.

On notera enfin qu'il serait possible de générer deux faisceaux ionisants non parallèles au moyen de deux réticules (par exemple deux fentes distinctes mémorisées dans une même plaque métallique) à partir d'une source radioactive unique pour mettre en oeuvre l'invention, en utilisant comme précédemment deux détecteurs disposés face aux deux faisceaux et déplaçables en synchronisme avec la source et les réticules.

## Revendications

1. Procédé d'imagerie radiographique pour la reconstruction tridimensionnelle à faible dose d'irradiation, adapté pour calculer un modèle à trois dimensions d'au moins un objet prédéterminé (20) à observer dans un champ d'observation, (4) dans lequel :

   1) on repère sur au moins deux images radiographiques des lignes de contour correspondant à des limites de l'objet et/ou à des lignes de plus grande densité optique à l'intérieur desdites limites,
   2) on crée un modèle générique recalé en adaptant la taille d'un modèle générique correspondant audit objet et la position dudit modèle générique pour que les projections respectives du modèle générique recalé à partir de deux sources radioactives (5, 11) correspondent sensiblement aux deux images radiographiques,
   3) on sélectionne des repères du modèle générique recalé dont les projections sur au moins une des images radiographiques à partir de la source radiographique correspondante sont les plus proches des lignes de contour repérées au cours de l'étape 1),
   4) on définit une surface enveloppe formée par des rayons issus de chaque source radiographique et ayant contribué à générer lesdites lignes de contour,
   5) on détermine certains repères du modèle générique recalé correspondant à des surfaces dudit modèle générique recalé, qui sont tangentes auxdites surfaces enveloppes,
   6) on détermine la position géométrique de chaque repère déterminé à l'étape 5) par projection dudit repère sur la surface enveloppe correspondante,
   7) on calcule la forme à trois dimensions d'un modèle représentant ledit objet par déformation du modèle générique recalé de façon à maintenir la coïncidence des repères du modèle générique déformé avec la position spatiale précédemment déterminée des repères et de façon que ledit modèle calculé suive une forme la plus proche possible d'une isométrie du modèle générique.

2. Procédé selon la revendication 1, dans lequel au cours de l'étape 7) on travaille sur l'ensemble des points du modèle générique.

3. Procédé selon la revendication 1 ou 2, ce procédé comprenant en outre l'étape de a) prendre au moins deux images radiographiques à deux dimensions du champ d'observation, respectivement selon deux directions de prise de vue (7, 13) non parallèles.

4. Procédé selon la revendication 3 dans lequel, au cours de l'étape a), les deux images radiographiques sont prises simultanément, par balayage, en déplaçant en synchronisme, dans une même direction de translation (3a) non parallèle aux directions de prises de vues, au moins une source radioactive (5, 11) émettant deux faisceaux de rayons ionisants (10, 16) respectivement dans les deux directions de prise de vue (7, 13).

5. Procédé selon l'une quelconque des revendications 3 ou 4, dans lequel les deux directions de prise de vue (7, 13) sont perpendiculaires l'une à l'autre.

6. Procédé selon la revendication 5, dans lequel on fait émettre par chacune des sources radioactives (5, 11) un faisceau de rayonnements ionisants (10, 16) dans un plan perpendiculaire à la direction de translation (3a).

7. Procédé selon l'une quelconque des revendications 3 à 6, dans lequel les deux faisceaux de rayons ionisants (10, 16) sont émis respectivement par deux sources radioactives (5, 11).

8. Produit programme d'ordinateur adapté pour mettre en oeuvre le procédé selon l'une des revendications 1 à 7, lorsqu'il est mis en oeuvre sur une machine programmable.

## Patentansprüche

1. Verfahren zur Röntgenbildgebung für die dreidimensionale Rekonstruktion mit niedriger Strahlungsdosis, welches geeignet ist, ein dreidimensionales Modell wenigstens eines vorbestimmten Objekts (20) zu berechnen, das in einem Betrachtungsfeld (4) zu betrachten ist, wobei:

1) auf wenigstens zwei Röntgenbildern Umrisslinien markiert werden, die den Grenzen des Objekts entsprechen, und/oder Linien größerer optischer Dichte im Inneren dieser Grenzen entsprechen,

2) ein rekalibriertes generisches Modell erzeugt wird, indem die Größe eines dem Objekt entsprechenden generischen Modells und die Position dieses generischen Modells so angepasst werden, dass die jeweiligen Projektionen des rekalibrierten generischen Modells von zwei radioaktiven Quellen (5, 11) aus im Wesentlichen den zwei Röntgenbildern entsprechen,

3) Bezugspunkte des rekalibrierten generischen Modells gewählt werden, deren Projektionen auf wenigstens eines der Röntgenbilder von der entsprechenden Röntgenquelle aus den Umrisslinien, die in Schritt 1) markiert wurden, am nächsten sind,

4) eine Hüllfläche definiert wird, die von Strahlen gebildet wird, die von jeder Röntgenquelle ausgehen, und die dazu beitragen, die Umrisslinien zu erzeugen,

5) gewisse Bezugspunkte des rekalibrierten generischen Modells bestimmt werden, die den Flächen des rekalibrierten generischen Modells entsprechen, welche tangential zu den Hüllflächen sind,

6) die geometrische Position jedes in Schritt 5) bestimmten Bezugspunktes durch Projektion dieses Bezugspunktes auf die entsprechende Hüllfläche bestimmt wird,

7) die dreidimensionale Form eines Modells, welches das Objekt repräsentiert, durch Verformung des rekalibrierten generischen Modells so berechnet wird, dass die Übereinstimmung der Bezugspunkte des verformten generischen Modells mit der zuvor bestimmten räumlichen Position der Bezugspunkte aufrechterhalten wird, und derart, dass das berechnete Modell einer Form folgt, die einer Isometrie des generischen Modells möglichst nahe kommt.

**2.** Verfahren nach Anspruch 1, wobei im Schritt 7) mit der Gesamtheit der Punkte des generischen Modells gearbeitet wird.

**3.** Verfahren nach Anspruch 1 oder 2, wobei dieses Verfahren außerdem folgenden Schritt umfasst: a) wenigstens zwei zweidimensionale Röntgenbilder des Betrachtungsfeldes in zwei nicht parallelen Aufnahmerichtungen (7, 13) aufzunehmen.

**4.** Verfahren nach Anspruch 3, wobei im Schritt a) die zwei Röntgenbilder gleichzeitig durch Abtasten aufgenommen werden, indem wenigstens eine radioaktive Quelle (5, 11), die zwei ionisierenden Strahlenbündel (10, 16) in den zwei jeweiligen Aufnahmerichtungen (7, 13) aussendet, synchron in ein und derselben Translationsrichtung (3a) verschoben wird, die zu den Aufnahmerichtungen nicht parallel ist.

**5.** Verfahren nach einem der Ansprüche 3 oder 4, wobei die zwei Aufnahmerichtungen (7, 13) zueinander senkrecht sind.

**6.** Verfahren nach Anspruch 5, wobei bewirkt wird, dass durch jede der radioaktiven Quellen (5, 11) ein ionisierendes Strahlenbündel (10, 16) in einer zu der Translationsrichtung (3a) senkrechten Ebene ausgesendet wird.

**7.** Verfahren nach einem der Ansprüche 3 bis 6, wobei die zwei ionisierenden Strahlenbündel (10, 16) von zwei jeweiligen radioaktiven Quellen (5, 11) ausgesendet werden.

**8.** Computerprogrammprodukt, welches dafür eingerichtet ist, das Verfahren nach einem der Ansprüche 1 bis 7 durchzuführen, wenn es auf einer programmierbaren Maschine ausgeführt wird.

**Claims**

**1.** Method of radiographic imaging for producing a three-dimensional reconstruction with a low dose of irradiation, configured to calculate a three-dimensional model of at least one predetermined object (20) to be observed in an observation field (4), wherein:

1) contour lines corresponding to limits of the object and/or to lines of a higher optical density within said limits are marked on at least two radiographic images,

2) a calibrated generic model is created by adapting the size of a generic model corresponding to said object and the position of said generic model so that the respective projections of the generic model calibrated using two radioactive sources (5, 11) substantially correspond to the two radiographic images,

3) markers of the calibrated generic model whose projections on at least one of the images taken using the corresponding radiographic source are closest to the contour lines marked during step 1) are selected,

4) an envelope surface is defined, formed by rays emitted from each radiographic source and having contributed to generating said contour lines,

5) certain markers of the calibrated generic model corresponding to surfaces of said calibrated generic model that are tangential to said envelope surfaces are determined,

6) the geometric position of each marker determined in step 5) is determined by projecting said marker onto the corresponding envelope surface,

7) the three-dimensional shape of a model representing said object is calculated by deforming the calibrated generic model so that markers of the deformed generic model remain coincident with the previously determined spatial position of the markers and so that said calculated model conforms to a shape that is as close as possible to an isometry of the generic model.

2. Method as claimed in claim 1, wherein work is carried out on all the points of the generic model during step 7).

3. Method as claimed in claim 1 or 2, this method further comprising the step a) of taking at least two two-dimensional radiographic images of the observation field respectively in two shooting directions (7, 13) that are not parallel.

4. Method as claimed in claim 3, wherein during step a), the two radiographic images are taken simultaneously by scanning, by displacing, in synchronism, in a same direction of translation (3a) that is not parallel with the shooting directions, at least one radioactive source (5, 11) emitting two beams (10, 16) of ionising radiation respectively in the two shooting directions (7, 13).

5. Method as claimed in any one of claims 3 or 4, wherein the two shooting directions (7, 13) are perpendicular to one another.

6. Method as claimed in claim 5, wherein a beam (10, 16) of ionising radiation is emitted by each of the radioactive sources (5, 11) in a plane perpendicular to the direction of translation (3a).

7. Method as claimed in any one of claims 3 to 6, wherein the two beams (10, 16) of ionising radiation are emitted respectively by two radioactive sources (5, 11).

8. Computer programme product configured to implement the method as claimed in one of claims 1 to 7 when run on a programmable machine.

FIG.1.

FIG.2.

FIG.3.

FIG.4.

FIG.5.

EP 2 309 462 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2749402 A

- FR 2754068 A

**Littérature non-brevet citée dans la description**

- **BDEL-AZIZ et al.** Direct linear transformation from comparator coordinates into object space coordinates in close range photogrammetry. *Proc. ASP/UI Symp. Close Range Photogrammetry,* 1971
- Rational design for close range photogrammetry. **MARZAN.** PhD thesis, Department of Civil Engineering. University of Illinois, 1976

- **ANDRÉ et al.** Optimized vertical stereo base radiographie setup for the clinical three-dimensional reconstruction of the human spine. *J. Biomech.,* 1994, vol. 27, 1023-1035
- **TROCHU.** A contouring program based on dual kriging interpolation. *Eng. Comput.,* 1993, vol. 9, 160-177
- **MITTON et al.** *Medical & Biological Engineering & Computing,* 2000, vol. 38, 133-139